# EUROPÄISCHE PATENTSCHRIFT

(11) **EP 2 438 975 B1**
(45) Veröffentlichungstag und Bekanntmachung des Hinweises auf die Patenterteilung: **23.04.2014**
(21) Anmeldenummer: 10013451.9
(22) Anmeldetag: 08.10.2010
(51) Int. Cl.: B01D 53/04

(54) **Verfahren zur Abtrennung von Kohlendioxid in Biogasanlagen**
Method for separating carbon dioxide in biogas systems
Procédé de séparation du dioxyde de carbone dans des installations de biogaz

(43) Veröffentlichungstag der Anmeldung: 11.04.2012
(73) Patentinhaber: Astrium GmbH, 82024 Taufkirchen (DE)
(72) Erfinder: Carsten, Matthias, Dr., 88046 Friedrichshafen (DE); Raatschen, Willigert, Dr., 88090 Immenstaad (DE); Brodt, Karlheinz, 88090 Immenstaad (DE); Lucas, Joachim, Dr., 88634 Großschönach (DE)
(74) Vertreter: Daub, Thomas

(56) Entgegenhaltungen:
- WO-A1-98/58726
- WO-A2-2008/072215
- DE-C1- 19 830 470
- GB-A- 2 020 191
- US-A- 5 389 125
- US-A- 5 797 979
- RUTHVEN D M ED - RUTHVEN D M: "Principles of Adsorption and Adsorption Processes, Adsorption Separation Processes, I. Cyclic Batch Systems", 1 January 1984 (1984-01-01), PRINCIPLES OF ADSORPTION AND ADSORPTION PROCESSES, JOHN WILEY & SONS, NEW YORK, PAGE(S) 336 - 343, XP002640253, ISBN: 978-0-471-86606-0

## Beschreibung

Die Erfindung betrifft ein Verfahren zur Abtrennung von Kohlendioxid in Biogasanlagen gemäß den Merkmalen des Patentanspruchs 1.

Für die Prozessgase in einer Biogasanlage wird zunächst folgendes definiert:
- Rohbiogas: Prozessgas, welches aus dem Fermenter entnommen wird.
- Aufbereitetes Rohbiogas: Rohbiogas, welches bereits eine Biogasentschwefelung und eine Gastrocknung durchlaufen hat, jedoch noch keine CO₂-Abtrennungsstufe.
- Biogas: Prozessgas, das nach der CO₂-Abtrennung überwiegend aus Biomethan besteht. Das Biogas kann nach entsprechender Konditionierung und Komprimierung in das Erdgasnetz eingespeist werden.

Es ist bekannt, Rohbiogas aus fermentativer Erzeugung aufzubereiten und Biomethan als Biogas zu gewinnen. Das Rohbiogas enthält je nach Substratzusammensetzung der Biogasanlage Methankonzentrationen von ca. 40 - 75 Vol.-% und Kohlendioxid in einem Konzentrationsbereich von ca. 25 bis 55 Vol.-%. Das Rohbiogas ist bei der Fermentationstemperatur mit Wasserdampf gesättigt und kann zusätzlich Minorkomponenten aufweisen. Die Aufbereitung des Rohbiogases umfasst daher zumeist folgende drei Verfahrensschritte, die Biogasentschwefelung, die Gastrocknung und die CO₂-Abtrennung. Das Biogas kann im Anschluss nach entsprechender Konditionierung und Komprimierung in das Erdgasnetz eingespeist werden.

Für die CO₂-Abtrennung sind adsorptive Abtrennverfahren unter Einsatz von Zeolithen (WO09/58726 oder WO2008/072215) oder Kohlenstoffmolekularsieben sowie absorptive Abtrennverfahren, wie die physikalische Wäsche mittels Wasser oder Genosorb^{®} oder die chemische Wäsche mittels MEA, DEA oder MDEA, bekannt. Für die genannten Verfahren gilt, dass je nach Einsatz des Ad- oder Absorbers entweder bei hohem Druck von 4 - 7 bar das CO₂ vom Methan abgetrennt und bei niedrigem Druck eine Regeneration des Ad- oder Absorbers nachgeschaltet ist (Druckwechselverfahren) oder dass bei niedriger Temperatur das CO₂ gebunden und bei hoher Temperatur der Ad- oder Absorber regeneriert wird (Temperaturwechselverfahren). Einen Überblick über die Vielzahl von unterschiedlichen Regenerationsverfahren ist beispielsweise dem Buch "Principles of Adsorption and Adsorption Processes" von Douglas M. Ruthven zu entnehmen. Eine Regeneration von adsorbiertem CO₂ durch Spielen mit vorgewärmtem Inertgas bei moderaten Temperaturen oder mittels Anlegen eines Vakuums ist aus US 5,797,979 bekannt. Aus der US-Patentanmeldung US 5,389,125 ist ein Verfahren zur Regeneration eines Aktivkohleadsorbers zur Abtrennung flüchtiger organischer Verbindung aus Luft bekannt, bei dem der Adsorber unter hohen Temperaturen mit Stickstoff als inertem Spülgas, das in einem geschlossenen Spülgaskreislauf über den Adsorber geleitet wird, regeneriert wird. In dem geschlossenen Spülgaskreislauf werden die desorbierten flüchtigen organischen Verbindungen mit einem Kondensator aus dem inertem Spülgas abgetrennt. Die genannten Druck- sowie Temperaturwechselverfahren sind energieaufwendig.

Aufgabe der Erfindung ist es, ein verbessertes Verfahren zur Abtrennung von Kohlendioxid in Biogasanlagen anzugeben. Diese Aufgabe wird durch das Verfahren gemäß den Merkmalen des geltenden Patentanspruchs 1 gelöst. Vorteilhafte Ausgestaltungen der Erfindung sind Gegenstand von Unteransprüchen.

Diese Aufgabe wird gelöst durch ein Verfahren, welches eine Adsorptionsphase zur Abtrennung von Kohlendioxid aus aufbereitetem Rohbiogas sowie eine Regenerationsphase umfasst. In der Adsorptionsphase wird aufbereitetes Rohbiogas bei Umgebungsdruck durch einen Adsorber aus zur Adsorption von Kohlendioxid geeigneten Adsorberharz geleitet und in der Regenerationsphase der Adsorber mit einem Spülgas bei Temperaturen zwischen 20 - 100°C sowie Umgebungsdruck regeneriert. Vor dem Abtrennverfahren des Kohlendioxids wird das Rohbiogas aufbereitet, wobei das Rohbiogas entschwefelt und getrocknet wird und Minorkomponenten entfernt werden. Zur Vermeidung von Verunreinigungen im Biogas zu Beginn der Adsorptionsphase wird ein Spülprozess des Adsorbers mit aufbereitetem Rohbiogas durchgeführt, wobei der Spülprozess zeitlich über eine Anzahl von Ventilen und einen Sensor gesteuert wird.

Die Regenerationszeit der Regenerationsphase kann durch die Prozessgrößen Temperatur und Volumenstrom des während der Regeneration verwendeten Spülgases, z.B. Luft oder Inertgas, beeinflusst werden. Die Prozessgrößen werden dabei zweckmäßig so gewählt, dass eine möglichst vollständige Regeneration des Adsorbermaterials erzielt wird und gleichzeitig der Energieaufwand für die Regeneration moderat ausfällt.

Die Erfindung sowie vorteilhafte Ausgestaltungen der Erfindung werden in Zeichnungen näher erläutert. Es zeigen:
- Fig. 1: eine schematisch dargestellte Vorrichtung zur Durchführung des erfindungsgemäßen Verfahrens
- Fig. 2: einen einzelnen Adsorber aus Fig. 1.

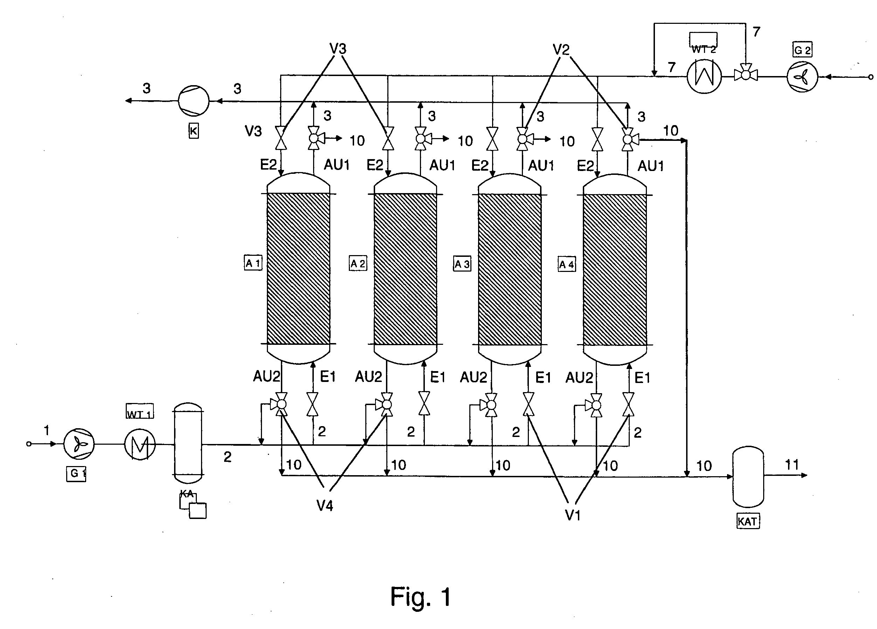

Fig. 1 zeigt eine Vorrichtung zur Durchführung des erfindungsgemäßen Verfahrens mit vier Adsorbern A1 bis A4. Die Zahl der Adsorber kann je nach Anlagengröße variiert werden.

Zweckmäßig befinden sich im Prozess zeitgleich zum einen mindestens ein oder mehrere Adsorber in der Adsorptionsphase und werden dabei vom aufbereitetem Rohbiogas 2 durchströmt und zum anderen mindestens ein oder mehrere Adsorber in der Regenerationsphase und werden dabei mit einem Spülgas 7 beaufschlagt. Das Spülgas 7 kann dazu mit einem Gebläse G2 der Prozessanlage zugeführt werden und mit einem zusätzlichen Wärmetauscher WT2 moderat vorgewärmt werden.

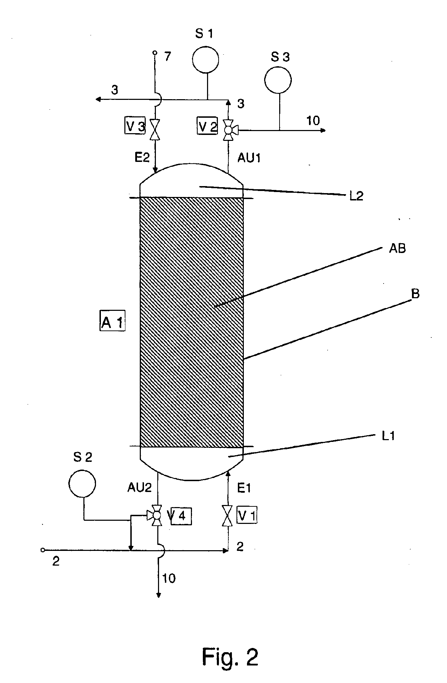

Anhand der Fig. 1 und 2 wird im Folgenden die Adsorptionsphase erläutert.

In der Adsorptionsphase wird entschwefeltes Rohbiogas 1 mit einem Gebläse G1 den Adsorbern A1 bis A4 zugeführt. Dabei durchläuft das entschwefelte Rohbiogas 1 zunächst zur Gastrocknung einen Wärmetauscher WT1 mit Kondensatabscheider KA. Im Anschluss kann das Gas in einer Aktivkohleeinheit (in Fig. 1 nicht dargestellt) von Minorkomponenten, wie Kohlenwasserstoffen, gereinigt werden. Anschließend wird das aufbereitete Rohbiogas den Adsorbern A1 bis A4 über den Eingang E1 zugeführt.

Während der Adsorptionsphase durchläuft das aufbereitete Rohbiogas 2 das Adsorberbett AB (Fig. 2) und verlässt den Adsorberbehälter B als Biogas über den Ausgang AU1. Zu Beginn der Adsorptionsphase ist das Ventil V1 geöffnet. Die Stellung des Ventils V2 ist so gewählt, dass das Biogas 3, welches nach Durchlaufen des Adsorbers eine hohe Reinheit an Biomethan aufweist, einem Kompressor K nach Fig. 1 und nachfolgend dem Erdgasnetz zugeführt werden kann. Die Ventil V3 und V4 sind in der Adsorptionsphase geschlossen.

Im Biogas 3 wird mit dem Sensor S1 die CO₂ Konzentration während der Adsorption nach Fig. 2 überwacht und der Adsorptionsprozess gestoppt, wenn eine vorgegebene CO₂-Verunreinigung im Biogas 3 nach Durchlaufen des Adsorbers gemessen wird. Zum Ende des Adsorptionsprozesses werden die Ventile V1 und V2 geschlossen.

Im Weiteren wird anhand der Fig. 1 und 2 die Regenerationsphase erläutert.

Nach der Adsorptionsphase wird das mit CO₂ beladene Adsorberbett AB regeneriert. Hierzu kann zunächst Inertgas und im Anschluss Luft als Spülgas 7 in das Adsorberbett AB über den Eingang E2 und durch das geöffnete Ventil V3 geleitet werden (Fig. 1 und 2). Die Luft kann moderat mittels des Wärmetauschers WT2 vorgewärmt werden.

Um während der Regenerationsphase Methanverluste zu minimieren, wird zu Beginn der Regeneration die Stellung des Ventils V4 so gewählt, dass das zu Beginn der Regenerationsphase den Adsorber A1 bis A4 verlassende Gas, welches zu Beginn der Regeneration noch hohe Anteile an Methan enthält, dem aufbereiteten Rohbiogas 2 zurückgeführt werden kann. Das Methan kann dann in einem anderen Adsorber, welches sich zeitgleich in der Adsorption befindet, als Biogas 3 gewonnen werden.

Im Prozessgas am Austritt AU2 wird nach Durchlaufen des Adsorberbetts AB mit dem Sensor S2 die Methankonzentration ermittelt. Unterschreitet die gemessene Methankonzentration einen vorgegebenen Wert, wird die Ventilstellung V4 so gewählt, dass im Folgenden Luft mit dem desorbierenden CO₂ während der Regenerationsphase als Abgas 10 an die Umgebung 11 abgeführt wird. Das Abgas 10, welches noch geringe Mengen an Methan enthält, wird zusätzlich einem katalytischen Brenner KAT zugeführt. Die Abwärme des katalytischen Brenners KAT wird zur Wirkungsgradsteigerung in einer Biogasanlage genutzt werden.

Nach einer vorgegebenen Zeit wird die Regenerationsphase mit vorgewärmter Luft 7 beendet und es kann Umgebungslauft am Wärmetauscher WT2 als Spülgas 7 vorbeigeführt (Fig. 1) und das Adsorberbett AB vor Beginn der nächsten Adsorptionsphase abgekühlt werden. Das Adsorberbett AB kann zusätzlich mit einem Inertgas als Spülgas 7 geflutet werden. Die Ventile V3 und V4 werden am Ende der Regenerationsphase geschlossen.

Im Anschluss an die Regeneration wird mit der nächsten Adsorptionsphase begonnen. Es wird dazu erneut das Ventil V1 geöffnet und das aufbereitete Rohbiogas 2 wird dem regenerierten Adsorberbett AB zugeführt. Zu Beginn der Adsorptionsphase wird das Ventil V2 dabei so geschaltet, dass die sich anfänglich noch im Adsorberbehälter B befindliche Luft oder Inertgas der vorangegangenen Regenerationsphase als Abgas 10 abgeführt werden kann und somit Gasverunreinigungen im Biogas 3 minimiert werden. Dabei wird die Methankonzentration im Abgas 10 am Ausgang AU1 des Adsorbers mit dem Sensor S3 überwacht (Fig. 2). Erreicht die gemessene Methankonzentration am Sensor S3 einen vorgegebenen Wert, wird das Ventil V2 umgeschaltet, so dass im Weiteren das aus dem Ausgang AU1 des Adsorbers strömende Gas als Biogas 3 mit hoher Methanreinheit weiterverwendet werden kann.

Das erfindungsgemäße drucklose Verfahren verspricht, unter Verwendung eins CO₂-Adsorberharzes, die Vorteile einer hohen Produktreinheit und Biogasausbeute in Analogie zu den bekannten chemischen Waschverfahren. Durch die Regeneration mit Luft bei moderaten Temperaturen kann zusätzlich der Energieaufwand des Verfahrens deutlich geringer ausfallen, als bei bereits bekannten CO₂-Abtrenntverfahren.

Die Geometrie des Adsorberbehälters B kann frei gewählt werden und unterliegt nicht den Restriktionen, wie sie sich durch eine Druck- oder Temperaturwechselbelastung ergeben. Der Adsorberbehälter B wird zweckmäßig zylindrisch mit möglichst großem Bettdurchmesser ausgelegt, um niedrige Strömungsgeschwindigkeiten im Adsorberbett AB zu erzielen und die Druckverluste der Anlage zu minimieren. Die Höhe des Adsorberbetts AB kann so dimensioniert werden, dass eine scharfe Trennung von CO₂ und Methan am Ausgang AU1 des Adsorbers A1 bis A4 erzielt wird.

Das Wandmaterial des Behälters B für das Adsorberharz ist frei wählbar (z.B. Metall, Kunststoff) und wird nach wirtschaftlichen und verfahrenstechnischen Gesichtspunkten ausgewählt.

Oberhalb und unterhalb des Adsorberbetts AB befindest sich ein Leerraum L1, L2 im Adsorberbehälter B, um eine geringfügige Quellung des Adsorberharzes zu tolerieren und eine gleichmäßige Strömungsverteilung im Behälter B und im Adsorberbett AB zu gewährleisten. Ebenfalls ist eine Vorrichtung zur Strömungsverteilung am Behältereingang und Ausgang möglich (nicht dargestellt).

Weiterhin kann der Adsorber mit einer Vorrichtung (nicht dargestellt) versehen werden, mit der die während der Adsorption frei werdende Adsorptionswärme gewonnen und in den Biogasprozess zurückgeführt wird.

## Patentansprüche

1. Verfahren zur Abtrennung von Kohlendioxid in Biogasanlagen umfassend eine Adsorptionsphase, in welcher das Kohlendioxid in einem Adsorber (A1-A4) aus aufbereitetem Rohbiogas (2) abgetrennt und das gewonnene Biomethan als Biogas (3) einem Erdgasnetz zugeführt wird und
eine Regenerationsphase, in welcher der Adsorber (A1-A4) regeneriert und das abgetrennte Kohlendioxid der Umgebungsluft oder einem CO₂-Speicher zugeführt wird,
wobei Rohbiogas (1) vor dem Abtrennverfahren des Kohlendioxids aufbereitet wird, wobei das Rohbiogas (1) entschwefelt und getrocknet wird und Minorkomponenten entfernt werden können,
wobei in der Adsorptionsphaseaufbereitetes Rohbiogas (2) bei Umgebungsdruck durch einen Adsorber (A1-A4) aus zur Adsorption von Kohlendioxid geeignetem Adsorberharz geleitet und
in der Regenerationsphase der Adsorber (A1-A4) mit Luft oder Inertgas als Spülgas (7) bei Temperaturen zwischen 20 - 100°C sowie Umgebungsdruck regeneriert wird,
wobei
das Spülgas (7) vorgewärmt wird, am Ende der Regenerationsphase der Adsorber (A1-A4) mittels Spülung mit Umgebungsluft abgekühlt wird und zur Vermeidung von Verunreinigungen im Biogas (3) zu Beginn der Adsorptionsphase ein Spülprozess des regenerierten Adsorbers (A1-A4) mit aufbereitetem Rohbiogas (2) durchgeführt wird, bei dem Spülgas (7) der vorhergehenden Regenerationsphase als Abgas (10) abgeführt wird, wobei der Spülprozess zeitlich über eine Anzahl von Ventilen (V1-V4) und einen Sensor (S3) gesteuert wird und
während der Regenerationsphase Spülgas mit desorbierendem CO₂ als Abgas (10), welches noch geringe Mengen an Methan enthält, einem katalytischen Brenner (KAT) zugeführt wird, dessen Abwärme zur Wirkungsgradsteigerung der Biogasanlage genutzt wird.

2. Verfahren nach Anspruch 1,
**dadurch gekennzeichnet, dass**
die während der Adsorptionsphase frei werdende Adsorptionswärme einem Wärmespeicher oder Wärmeüberträger zugeführt wird.

3. Verfahren nach einem der vorangehenden Ansprüche,
**dadurch gekennzeichnet, dass**
während der Adsorptionsphase zur Vermeidung von CO₂-Verunreinlgungen im Biogas (3) die CO₂-Konzentration an einem Ausgang (AU1) des Adsorbers (A1-A4) mit einem Sensor (S1, S2, S3) überwacht und die Adsorptionszeit über die CO₂-Messung im Biogas (3) durch eine Anzahl von Ventilen (V1-V4) am Ein- und Ausgang (AU1) des Adsorbers (A1-A4) geregelt wird.

4. Verfahren nach einem der vorangehenden Ansprüche,
**dadurch gekennzeichnet, dass**
in der Regenerationsphase ein Spülgas (7) durch den Adsorber (A1-A4) geleitet und der Umgebungsluft zugeführt wird, wobei zur Vermeidung von Methanverlusten zu Beginn einer Regenerationsphase das sich im Adsorber (A1-A4) befindliche Gas dem Rohbiogas (2) zugeführt wird.

## Claims

1. Method for separating off carbon dioxide in biogas plants, comprising
an adsorption phase in which the carbon dioxide is separated off out of processed crude biogas (2) in an adsorber (A1-A4) and the biomethane obtained is fed as biogas (3) to a natural gas grid and
a regeneration phase in which the adsorber (A1-A4) is regenerated and the separated-off carbon dioxide is fed to the ambient air or to a CO₂ store,
wherein crude biogas (1) is processed before the carbon dioxide is separated off, wherein the crude biogas (1) is desulfurized and dried and minor components can be removed,
wherein, in the adsorption phase, processed crude biogas (2) is passed at ambient pressure through an adsorber (A1-A4) made of adsorber resin suitable for the adsorption of carbon dioxide, and
in the regeneration phase the adsorber (A1-A4) is regenerated with air or inert gas as purge gas (7) at temperatures between 20°C and 100°C and at ambient pressure,
wherein
the purge gas (7) is preheated, the adsorber (A1-A4) is cooled at the end of the regeneration phase by purging with ambient air and, to avoid impurities in the biogas (3) at the start of the adsorption phase, a purge process is carried out on the regenerated adsorber (A1-A4) using processed crude biogas (2), in which purge gas (7) of the preceding regeneration phase is withdrawn as off-gas (10), wherein the purge process is controlled with respect to time via a number of valves (V1-V4) and a sensor (S3) and,
during the regeneration phase, purge gas containing desorbent CO₂ as off-gas (10), which still contains minor quantities of methane, is fed to a catalytic burner (KAT), the waste heat of which is utilised to increase the efficiency of the biogas plant.

2. Method according to Claim 1, **characterized in that** the adsorption heat released during the adsorption phase is fed to a heat store or heat exchanger.

3. Method according to any one of the preceding claims,
**characterized in that**, during the adsorption phase, to avoid CO₂ impurities in the biogas (3), the CO₂ concentration at an exit (AU1) of the adsorber (A1-A4) is monitored by a sensor (S1, S2, S3) and the adsorption time is controlled via the CO₂ measurement in the biogas (3) by a number of valves (V1-V4) at the intake and the exit (AU1) of the adsorber (A1-A4).

4. Method according to any one of the preceding claims,
**characterized in that** a purge gas (7) is conveyed through the adsorber (A1-A4) in the regeneration phase and fed to the ambient air, wherein, to avoid methane losses at the start of a regeneration phase, the gas situated in the adsorber (A1-A4) is fed to the crude biogas (2).

## Revendications

1. Procédé de séparation de dioxyde de carbone dans des installations de biogaz, comprenant
une phase d'adsorption, lors de laquelle le dioxyde de carbone est séparé dans un adsorbeur (A1-A4) d'un biogaz brut traité (2) et le biométhane obtenu est introduit en tant que biogaz (3) dans un réseau de gaz naturel, et
une phase de régénération, lors de laquelle l'adsorbeur (A1-A4) est régénéré et le dioxyde de carbone séparé est introduit dans l'air ambiant ou dans un réservoir de CO₂,
le biogaz brut (1) étant traité avant le procédé de séparation du dioxyde de carbone, alors que le biogaz brut (1) est désulfuré et séché et des composants mineurs peuvent être ôtés,
lors de la phase d'adsorption, le biogaz brut traité (2) étant conduit à pression ambiante au travers d'un adsorbeur (A1-A4) en résine d'adsorption appropriée pour l'adsorption de dioxyde de carbone, et lors de la phase de régénération, l'adsorbeur (A1-A4) étant régénéré avec de l'air ou un gaz inerte en tant que gaz de rinçage (7) à des températures comprises entre 20 et 100 °C et à pression ambiante,
dans lequel
le gaz de rinçage (7) est préchauffé, refroidi à la fin de la phase de régénération de l'adsorbeur (A1-A4) par rinçage avec de l'air ambiant et un procédé de rinçage de l'adsorbeur (A1-A4) régénéré avec du biogaz brut traité (2) est réalisé au début de la phase d'adsorption pour éviter les impuretés dans le biogaz (3), le gaz de rinçage (7) de la phase de régénération précédente étant déchargé en tant que gaz d'échappement (10), le procédé de rinçage étant commandé dans le temps par un nombre de vannes (V1-V4) et un capteur (S3), et pendant la phase de régénération, le gaz de rinçage contenant du CO₂ désorbant est introduit en tant que gaz d'échappement (10), qui contient encore de petites quantités de méthane, dans un brûleur catalytique (KAT), dont la chaleur d'échappement est utilisée pour augmenter l'efficacité de l'installation de biogaz.

2. Procédé selon la revendication 1, **caractérisé en ce que** la chaleur d'adsorption dégagée pendant la phase d'adsorption est introduite dans un accumulateur de chaleur ou un caloporteur.

3. Procédé selon l'une quelconque des revendications précédentes, **caractérisé en ce que**, pendant la phase d'adsorption, pour éviter les impuretés de CO₂ dans le biogaz (3), la concentration en CO₂ à une sortie (AU1) de l'adsorbeur (A1-A4) est surveillée avec un capteur (S1, S2, S3) et le temps d'adsorption est régulé par le biais de la mesure de CO₂ dans le biogaz (3) par un nombre de vannes (V1-V4) à l'entrée et à la sortie (AU1) de l'adsorbeur (A1-A4).

4. Procédé selon l'une quelconque des revendications précédentes, **caractérisé en ce que** lors de la phase de régénération, un gaz de rinçage (7) est conduit à travers de l'adsorbeur (A1-A4) et introduit dans l'air ambiant, le gaz qui se trouve dans l'adsorbeur (A1-A4) au début d'une phase de régénération étant introduit dans le biogaz brut (2) pour éviter les pertes de méthane.
